Europäisches Patentamt

European Patent Office

Office européen des brevets

(19)

(11) Publication number: **0 062 954**

**A1**

(12) # EUROPEAN PATENT APPLICATION

(21) Application number: **82200426.3**

(22) Date of filing: **07.04.82**

(51) Int. Cl.³: **G 01 N 33/44**

(30) Priority: **07.04.81 NL 8101699**

(43) Date of publication of application:
**20.10.82 Bulletin 82/42**

(84) Designated Contracting States:
**AT BE CH DE FR GB IT LI LU NL SE**

(71) Applicant: **STAMICARBON B.V.**
**Postbus 10**
**NL-6160 MC Geleen(NL)**

(72) Inventor: **Vroomen, Leonardus Johannes**
**van Veldekelaan 9**
**NL-6165 EG Geleen(NL)**

(72) Inventor: **Doornekamp, Johan Gerrit Friedrich**
**Dr. Nolenslaan 50**
**NL-6162 EX Geleen(NL)**

(74) Representative: **Van de Panne, Vitus Nicolaas et al,**
**Octrooibureau DSM Postbus 9**
**NL-6160 MA Geleen(NL)**

(54) **Device for the determination of the stress relaxation of elastomeric materials under compressive loads.**

(57) Device for the determination of the stress relaxation under compressive loads of elastomeric materials. A test specimen (48) of the elastomeric material is held compressed to a predetermined degree (e.g. 25%) in a specimen clamp (16) between two compressing elements (44, 45). A number of specimen clamps is placed by turns under a pressure rod (23), which each turn is pressed on the upper compressing element (44) and in this way subjects the test specimen to a small additional compression, which is limited by a spacer sleeve (24) coaxial with the pressure rod. The force required for this is measured by means of a force transducer (25) mounted in the pressure rod (23); the stress relaxation is the percent decrease of this force within a certain time, e.g. a week. The specimen clamps can be kept at a predetermined temperature during the test.

FIG 1

EP 0 062 954 A1

## DEVICE FOR THE DETERMINATION OF THE STRESS RELAXATION OF ELASTOMERIC MATERIALS UNDER COMPRESSIVE LOADS

The invention relates to a device for the determination of the stress relaxation of elastomeric materials under compressive loads, which device is provided which at least one specimen clamp with a first and a second compressing element, whose respective endfaces are parallel and between which a test specimen made of the material to be tested, two sides of which are parallel, can be compressed, and held compressed, in the direction normal to these sides and to a predetermined degree, the apparatus being in addition provided with means to exert pressure on one of the compressing elements so as to subject the compressed test specimen to a small additional compression, and with means to measure the force required for this.

Such a device is known from US Patent Specification No. 2,831,341. In this known device, the specimen clamp comprises two parallel plates (4, 16), with spacing blocks (18, 19) provided between them which can be clamped between the plates with four bolts (6-9) so that the distance between the plates is accurately known. One compressing element (14) is mounted in the bottom plate, the other (1) tests against the underside of the top plate with a flange (15) and can slide axially in an electrically insulated ball bushing (3). When during application of the load to the upper compressing element the pressure at which the specimen is clamped between the compressing elements is transgressed, the flange (15) is separated from the top plate, causing a sudden increase in the electrical resistance between the upper compressing element and the top plate, which is detected with a measuring circuit; at this instant, the load is measured.

This known device has the disadvantage that during measurement the load is continuously increased; the moment at which this load is to be measured is not accurately known beforehand; this results in a certain 'overshoot'. Moreover, the definition of the moment when the electrical contact between the compressing element and the top plate is perceptibly broken is not accurate enough. For these reasons, it is difficult to obtain accurate and consistent results when measuring with this known device.

The invention provides a device with which, in a simple way, accurate and consistent measuring values can be obtained. In addition, the invention provides a device with which several test specimens can be tested simultaneously, which offers a considerable saving of time.

The device according to the invention is characterized by limiting means which cooperate with the means to accomplish the additional compression and which prevent further compression when the additional compression has reached a predetermined value, preferably in such a way that the limiting means move along with the means to accomplish the additional compression and are arrested by the specimen clamp when the additional compression has reached the predetermined value, thus preventing further compression. Limiting is thus effected in a very simple manner. The limiting means can also be of a different nature, however; the accomplishment of the predetermined compression can for example be detected optically.

In a preferred embodiment of the device according to the invention, the specimen clamp comprises in essence a housing with a cylindrical bore constricted at one end by a collar, a first compressing element which can slide axially in said bore and which can rest against said collar, a second compressing element which can slide axially in said bore and which has been provided with a flange which can rest against the opposite end of the housing, it being possible to compress, and hold compressed, a test specimen between these two compressing elements, and a locknut to be screwed on the housing, with which said flange can be clamped to the housing; the means to subject the test specimen to a slight additional compression preferably consist in essence of a pressure rod provided with a force transducer, which can be pressed with its end-face against said first compressing element, and said limiting means preferably comprise in essence a spacer sleeve coaxial with this pressure rod, which can be pressed against the housing of the specimen clamp with its end-face, the distance between the end-face of the spacer sleeve and the end-face of the pressure rod being such that the displacement of the first compressing element by the pressure rod corresponds to the predetermined additional compression.

The invention will be explained with reference to the drawing, where a device according to the invention is represented schematically as a non-restricting example. In this drawing,

Fig. 1: shows a side elevation, partly in section, of the device;

Fig. 2: shows a more detailed side elevation, in section, of a specimen clamp;

Fig. 3: shows the encircled part indicated as III in Fig. 1 in more detail.

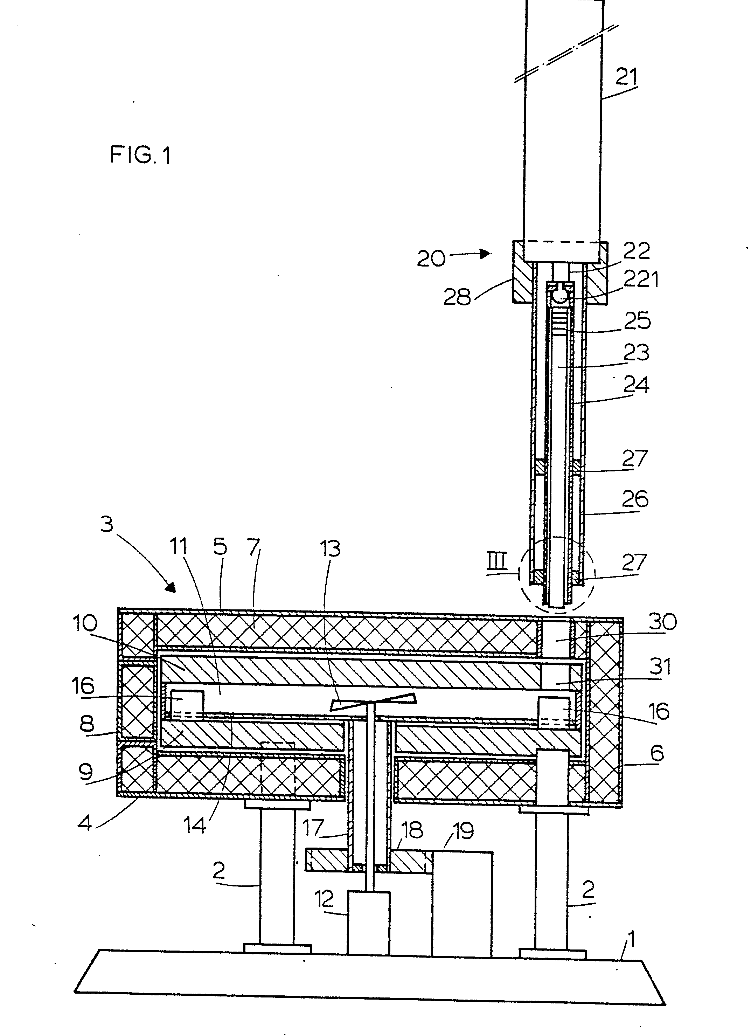

As schematically indicated in Fig. 1, the apparatus has been provided with a baseplate 1 supporting , on columns 2, a housing 3. The bottom 4, the upper wall 5 and the cylindrical side wall 6 of the housing 3 have been provided with a layer of heat-insulating material 7. A door 8, which has also been provided with heat-insulating material, provides access to the interior of the housing 3. In the housing 3, two thick round metal plates 9 and 10 have been placed, one some distance above the other, in which electric spiral heaters (omitted from the drawing) have been installed. The space 11 between plates 9 and 10 is the testing chamber proper; to promote uniform heating in chamber 11, a fan 13 driven by a motor 12 has been installed for circulation of the air therein.

In the bottom of chamber 11 is a conveyor disc 14, which has been provided with a number of openings (e.g. about 30) along its periphery, in which specimen clamps 16 can be placed. The conveyor disc 14 is mounted on a bushing 17 to whose other end a ratchet-wheel 18 has been fixed. The body formed by conveyor disc 14, bushing 17 and ratchet-wheel 18 can be rotated stepwise by means of a ratchet mechanism 19, the specimen clamps 16 being moved stepwise along the periphery of chamber 11. One of the positions successively occupied by every specimen clamp 16 is directly under the system indicated by 20, which comprises in essence a pneumatic or hydraulic cylinder 21 to whose piston rod 22 a pressure rod 23 and a spacer sleeve 24 coaxial therewith have been fitted by a ball and socket joint 221.

In the pressure rod 23 a force transducer 25 has been incorporated (suitable force transducers are commercially available). The spacer sleeve 24 is guided in a guide bushing 26 with guide rings 27; the pressure rod 23 and spacer sleeve 24 can be moved up and down by means of the pneumatic or hydraulic cylinder 21. The system 20 comprising parts 21 to 27 inclusive has been mounted in a bridge 28 which is perpendicular to the plane of the drawing of Fig. 1, and which has been mounted on two columns (omitted from the drawing), which in turn have been mounted on baseplate 1. Through opening 30 in the upper wall 5 of the housing 3 and opening 31 in plate 10, the pressure rod 23 and spacer sleeve 24 can be pressed on the specimen clamp 16 located under the openings.

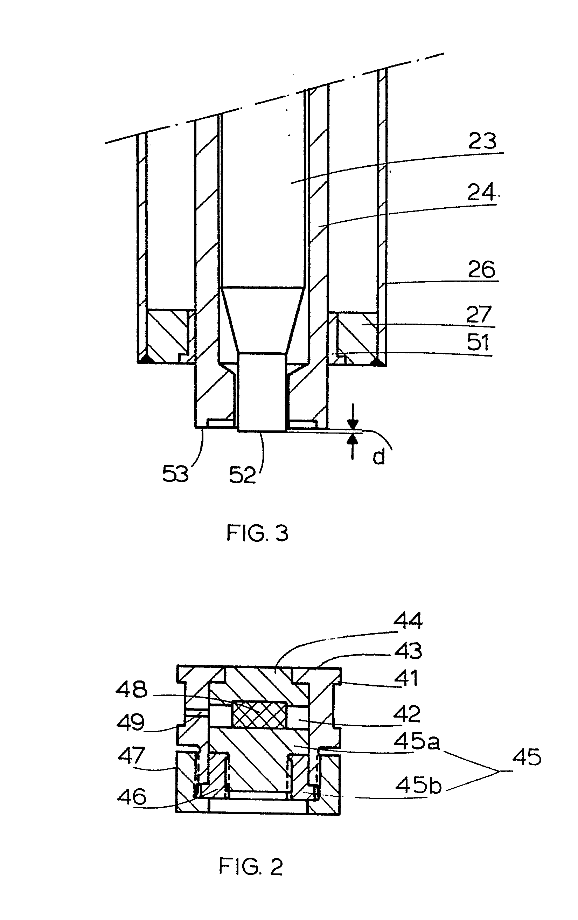

The construction of a specimen clamp 16 is specified in Fig. 2. The specimen clamp is composed of the following parts:

- a housing 41 with a cylindrical bore 42 constricted at the top by a collar 43;

- a first (upper) compressing element 44 which can slide axially in bore 42 and which can rest against collar 43; the top part of compressing element 44 has a smaller diameter than the bottom and extends into collar 43 in such a way that the top of the housing 41 and the top part of the compressing element 44 are precisely coplanar when compressing element 44 rests against collar 43;

- a second (lower) compressing element 45 which can slide axially in bore 42 and which comprises two parts 45a and 45b, one of which can be screwed into the other, so that the axial length of compressing element 45 is adjustable; the lower part 45b has been provided with a flange 46 which can rest against the underside of the housing 41;

- a locknut 47 to clamp flange 46 against housing 41.

A specimen to be tested 48 is compressed between the compressing elements 44 and 45, air being allowed to escape through vent hole 49. The degree of compression is usually 25 $\pm$ 2 %. The diameter of the test specimen 48, when it is not under load, is usually 13.0 $\pm$ 0.5 mm and the thickness 6.3 $\pm$ 0.3 mm. Parts 45a and 45b of compressing element 45, one of which can be screwed into the other, have been provided with a scale division on their circumference; by screwing the parts closer to each other or wider apart, the axial length of compressing element 45 can be accurately adapted to the thickness of the test specimen 48 so that it is always possible to realize a compression of 25 %.

The lower part of system 20, encircled at III in Fig. 1, is represented in more detail in Fig. 3. Parts 23, 24, 26 and 27 have been dealt with above; the guiding ring 27 has been provided with a bronze inner bushing 51. The end-face 52 of the pressure rod 23 is a distance d ahead of the end-face 53 of spacer sleeve 24, for example 0.03 mm. Now if pressure rod 23 and spacer sleeve 24 are jointly pressed on the specimen clamp 16 until the end-face 53 of spacer sleeve 24 is at rest against the upper face of the housing 41 of the specimen clamp 16, compressing element 44 is pressed into the housing along a distance d, and the test specimen 48 is subject to an additional compression d. The force required for this is measured with force transducer 25.

In order to be sure that for all specimen clamps the additional compression is d, the upper face of the housing 41 should always be exactly coplanar with the upper face of the part of compressing element 44 which extends into collar 43 when the compressing element is at rest against collar 43, as pointed out above. This can be accomplished in a simple manner by grinding these upper faces jointly during the fabrication process after the specimen clamp has been completely assembled. Also in other respects, a specimen clamp of the form indicated in Fig. 2 is simple to fabricate and therefore not very costly, which is important when larger numbers of specimen clamps are used.

For controlling the apparatus during a measuring cycle and for the processing of the measuring data, a microprocessor-based control and data-processing system is present, which has been omitted from the drawing. Such systems are known in the art.

A measuring cycle for testing a number of elastomeric materials – hereafter called 'rubber' for convenience –, proceeds as follows:

Of each rubber to be tested at least three cylindrical test specimens are made having a diameter of $13.0 \pm 0.5$ mm and a thickness of $6.3 \pm 0.3$ mm; the correct thickness is accurately measured. Next, each test specimen is placed in a specimen clamp according to Fig. 2 between the compressing elements 44 and 45, the housing with the compressing elements 44 and 45 and the test specimen 48 between them is compressed with an auxiliary device until flange 46 is arrested by the housing 41, and locknut 47 is screwed tight. By this procedure, the test specimen 48 is compressed to 75 % of its original thickness; as discussed above, the axial length of compressing element 45 can be adjusted for small differences in thickness which may occur, by slightly turning the screwable parts 45a and 45b with respect to each other before compressing.

The specimen clamps are now placed in the apparatus, in the openings of the conveyor disc 14, and the apparatus is closed and brought to the temperature of test. When the whole – the interior of the apparatus, the specimen clamps and the rubber discs therein which are to be tested - have reached the temperature of test, for example after 2 hours, a first measurement of the compression force is undertaken for all test specimens (force measured $= F_0$). After approximately 168 hours, the last measurement is done (force measured $= F_1$).

The stress relaxation under compressive load $R_t$ at a particular temperature t °C, of a particular rubber is then defined as

$$R_t = \frac{F_0 - F_1}{F_0} \times 100 \ \%$$

(the average of at least three test specimens).

If desired, intermediate measurements can be done, for example every 24 hours, to follow the test through its stages.

CLAIMS

1. Device for the determination of the stress relaxation of elastomeric materials under compressive loads, which device is provided with at least one specimen clamp with a first and a second compressing element whose respective end-faces are parallel and between which a test specimen made of the elastomeric material to be tested, two sides of which are parallel, can be compressed, and held compressed, in the direction normal to these sides, to a predetermined degree, the device being in addition provided with means to exert pressure on one of the compressing elements so as to subject the compressed test specimen to a small additional compression, and with means to measure the force required for this, the device being characterized by limiting means which cooperate with the means to accomplish the additional compression and which when the additional compression has reached a predetermined value prevent further compression.

2. Device according to claim 1, characterized in that the limiting means move along with the means for accomplishing the additional compression and are arrested by the specimen clamp when the additional compression has reached the predetermined value, thus preventing further compression.

3. Device according to claim 1 or 2, characterized in that the specimen clamp (16) comprises in essence a housing (41) with a cylindrical bore (42) constricted at one end by a collar (43), a first compressing element (44) which can slide axially in said bore and which can rest against said collar (43), a second compressing element (45) which can slide axially in said bore and which has been provided with a flange (46) which can rest against the opposite side of the housing (41), it being possible to compress, and hold compressed, a test specimen (48) between these two compressing elements, and a locknut (47) to be screwed on the housing (41), with which said flange (46) can be clamped against the housing (41).

4. Device according to claim 3, characterized in that the upper part of said first compressing element (44) has a smaller diameter than the lower part and extends into said collar (43), in such a way that the top face of the housing (41) is coplanar with the top face of the part of the compressing element (44) extending into the collar when this compressing element (44) rests against the collar (43).

5. Device according to any one of claims 2-4, characterized in that the means to subject the compressed test specimen (48) to a small additional compression consist in essence of a pressure rod (23) provided with a force transducer (25) which can be pressed against said first compressing element (44) with its end-face (52) and in that said limiting means comprise in essence a spacer sleeve (24) coaxial with this pressure rod (23), which can be pressed against the housing (41) of the specimen clamp with its end-face (53), the distance (d) between the end-face (52) of the pressure rod (23) and the end-face (53) of the spacer sleeve (24) being such that the displacement of the first compressing element (44) caused by the pressure rod (23) corresponds to the predetermined additional compression, there being in addition means (1, 2, 9) to support the specimen clamp (16) and means (21) to press the pressure rod and the spacer sleeve (24) thereon.

6. Device according to any one of claims 1-5, characterized in that the axial length of one of the compressing elements of the specimen clamp can be adjusted, to which end this compressing element (45) comprises two parts (45a, 45b) one of which can be screwed into the other.

7. Device according to claim 4, 5 or 6, characterized by a plurality of specimen clamps (16) and means to place these under the pressure rod (23) by turns.

8. Device according to claim 7, characterized in that the means for placing the specimen clamps (16) under the pressure rod (23) by turns comprise in essence a rotatable disc (14) which has been provided with recesses along its periphery, wherein the specimen clamps can be placed, and a drive mechanism (17, 18, 19) for stepwise rotation of this disc (14).

9. Device according to any one of the foregoing claims, characterized by means to keep the specimen clamps and the test specimens present therein at a predetermined temperature.

10. Device, substantially as described and explained with reference to the drawing.

FIG.1

23
24
26
27
51

d

53    52

FIG. 3

44
43
41
48
42
49
47    45a    45
46    45b

FIG. 2

**0062954**

**European Patent Office**

**EUROPEAN SEARCH REPORT**

Application number

EP 82 20 0426

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 529 223 (RUBBER AND PLASTICS RESEARCH ASSOCIATION) * page 3, claim 1; figure 1 * | 1 | G 01 N 33/44 |
| | --- | | |
| A | US-A-3 934 463 (A.D. VENDERJAGT) * first page * | 1 | |
| | --- | | |
| A | FR-A-2 046 445 (GOODYEAR) * page 71 * | 1 | |
| | --- | | |
| A | US-A-4 227 400 (J.F. NIENOW) * first page * | 1 | |
| | ----- | | |

| TECHNICAL FIELDS SEARCHED (Int. Cl. 3) |
|---|
| G 01 N 33/44 |
| G 01 N 3/40 |
| G 01 N 3/42 |

The present search report has been drawn up for all claims

| Place of search | Date of completion of the search | Examiner |
|---|---|---|
| THE HAGUE | 14-07-1982 | DUCHATELLIER M.A. |